Europäisches Patentamt

(19) European Patent Office

Office européen des brevets

(11) Publication number: **0 018 333**
**A2**

(12) # EUROPEAN PATENT APPLICATION

(21) Application number: **80850039.1**

(22) Date of filing: **26.03.80**

(51) Int. Cl.³: **C 12 N 11/02,** C 12 N 11/18,
C 12 P 7/50
// C12P11/00

(30) Priority: **27.03.79 SE 7900826**

(43) Date of publication of application: **29.10.80**
**Bulletin 80/22**

(84) Designated Contracting States: **AT DE FR GB IT NL SE**

(71) Applicant: **Brodelius, Peter, 8 B, Dag Hammarskjölds**
**väg, S-223 64 Lund (SE)**
Applicant: **Mosbach, Klaus, 31 Lackalänga,**
**S-244 02 Furulund (SE)**

(72) Inventor: **Brodelius, Peter, 8 B, Dag Hammarskjölds väg,**
**S-223 64 Lund (SE)**
Inventor: **Mosbach, Klaus, 31 Lackalänga,**
**S-244 02 Furulund (SE)**

(74) Representative: **Hansson, Sven A., AB STOKHOLMS**
**PATENTBYRA Zacco & Bruhn Box 3129,**
**S-103 62 Stockholm (SE)**

(54) **Immobilized microbial enzyme/cell systems, process for their production and their use in preparing alpha-keto acids from the corresponding amino acids.**

(57) According to the invention, α-keto acids are prepared from the corresponding amino acids by using a biocatalyst of a microbial origin, which has been immobilized in a polymeric matrix. The immobilized biocatalyst is effective and specific for the synthesis of α-keto acids from D-amino acids. The product can easily be separated from the particulate catalyst. In the synthesis reaction, hydrogen, peroxide is formed, which is decomposed by a catalyst which also is immobilized in the polymeric matrix. The invention can be used in continuous or discontinuous processes for the preparation of α-keto acids from the corresponding D,L- or D-amino acids.

EP 0 018 333 A2

The use of immobilized microbial enzyme/cell systems
for the preparation of α-keto acids from the
corresponding amino acids

The present invention refers to new catalysts for the preparation of α-keto acids from the corresponding amino acids. The invention further refers to a method for the preparation of the catalysts of the invention, and also to the use of these catalysts for the preparation of α-keto acids from the corresponding amino acids.

α-keto acids are used for the treatment of patients having a decreased kidney function, and also for testing the "liver clearance", and in other fields. At present, such acids are prepared by complicated synthetic processes, which comprise a multitude of steps and give relatively low yields.

By the present invention, the above disadvantages are removed. According to the invention, α-keto acids are prepared from the corresponding amino acids by the use of a biocatalyst for the transformation of amino acids to α-keto acids, said biocatalyst being bonded or immobilized in a polymeric matrix. As the immobilized biocatalyst is effective and specific for the synthesis of α-keto acids from the corresponding D-amino acids, the synthesis of the end product can be carried out at a very decreased cost.

The biocatalyst used may be a pure enzyme, a cell homogenate or whole cells of organisms which can perform the synthesis. As examples of such organisms can be mentioned <u>Trigonopsis variabilis</u>, <u>Candida tropicalis</u> and <u>Chlorella vulgaris</u>, but also other organisms are useful and are known to one skilled in the art. Especially Trigonopsis variabilis may be pointed out, which specifically oxidizes D-amino acids to the corresponding α-keto acids, while from racemic mixtures L-amino acids are also obtained.

The polymer in which the biocatalyst is bonded or immobilized preferably is a porous polymer, in the pores or network of which a biocatalyst is confined, adsorbed or covalently bonded. The polymer preferably is a polysaccharide, such as alginate, agar or carrageenan, an acrylic polymer, such as polyacryl amide, or a cross-linked polyamine. The crosslinked polyamine can preferably be an inert protein, such as albumin or gelatine, which has been cross-linked with a di- or polyaldehyde, such as glutaric aldehyde. Also polysaccharides, such as chitin, may be cross-linked with a di- or polyaldehyde.

In the synthesis process for the $\alpha$-keto acid, hydrogen peroxide is also formed, which may be decomposed by a further catalyst, which also is bonded or immobilized in the polymer matrix in the same way as the biocatalyst for the preparation of the $\alpha$-keto acids. The hydrogen peroxide decomposing catalyst may be an enzyme, especially then catalase or peroxide dismutase, and/or an organic or inorganic catalyst, especially then one or more metal oxides.

The catalyst of the invention may be prepared by mixing a solution or suspension of a polysaccharide, a polymerizable monomer or a polyamine with whole cells, a cell-free extract or a purified enzyme which contains the biocatalyst and, if desired, also with a hydrogen peroxide decomposing catalyst, after which the polymerizable monomer is polymerized, or the polymer is cross-linked, depending on the starting material for the polymer. When alginate or carrageenane is used as a polymer, this polymer may be cross-linked by contacting the solution or suspension of the polymer with a salt solution, preferably then of calcium or magnesium chloride. Acrylic monomers may be polymerized in a way known per se.

The polymer with the immobilized catalyst therein may then be recovered in a suitable way and be charged in a reactor of a suitable construction for continuous or discontinuous operation. In the preparation of α-keto acids from the corresponding amino acids, the polymer, which preferably is present in a particulate form, is contacted with a substrate solution which contains the appropriate amino acid in the D,L- or D-form, and the contact between the catalyst and the amino acid is maintained for a time sufficient to form the desired amount of the α-keto acid.

The invention is further illustrated by the following example:

Example: One gram of cells of Trigonopsis variabilis are mixed with 10 g of 2 per cent alginate solution and is immobilized by adding the mixture dropwise to a 0.1 M solution of $CaCl_2$. To the immobilized cells is subsequently added 50 ml of a 10 mM solution of D-methionine. After one hour, the amino acid has quantitatively and specifically been transformed into the corresponding α-keto acid, namely α-keto-γ-methiolbutyric acid.

-------------------

## CLAIMS

1. A catalyst for the preparation of α-keto acids from the corresponding amino acids, characterized in that it comprises particles of a preferably porous polymer, in the pores or network of which a biocatalyst is confined, adsorbed and/or covalently bonded.

2. The catalyst of claim 1, characterized in that the catalyst consists of whole cells, a cell-free extract or a purified enzyme of a microbial origin, preferably then from Trigonopsis variabilis.

3. The catalyst of claim 1, characterized in that the polymer is a polysaccharide, preferably alginate or carrageenan, an acrylic polymer or a cross-linked polyamine.

4. The catalyst of any of claims 1-3, characterized in that it also contains a confined, adsorbed and/or covalently bonded catalyst which decomposes hydrogen peroxide.

5. The catalyst of claim 4, characterized in that the hydrogen peroxide decomposing catalyst is an enzyme, preferably catalase or peroxide dismutase, and/or an organic or inorganic catalyst, preferably one or more metal oxides.

6. A process for the preparation of a catalyst for the preparation of α-keto acids from the corresponding amino acids in accordance with any of claims 1-5, characterzed in that a solution or suspension of a polysaccharide, a polymerizable monomer or a polyamine and whole cells, a cell-free extract or purified enzyme of a microbial origin and optionally a hydrogen peroxide decomposing catalyst is polymerized.

7. The process of claim 6, characterized in that a polyvalent polysaccharide, especially alginate or carrageenan, is polymerized by contacting the suspension or solution of the polysaccharide with a salt solution, especially then of calcium or magnesium chloride.

8. The process of claim 6, characterized in that an acrylic monomer is used as a polymerizable monomer.

9. The process of claim 6, characterized in that a polyamine, especially then an inert protein, such as albumin or gelatine, or a polysaccharide, such as chitin, is cross-linked with a di- or polyaldehyde, preferably then glutaric aldehyde.

10. The use of a catalyst in accordance with claim 1 for the preparation of $\alpha$-keto acids from the corresponding amino acids, wherein the polymer particles are contacted with substrate solutions which contain D,L- or D-amino acids, in a continuous or discontinuous reactor.

-----------------------------